# EUROPÄISCHE PATENTSCHRIFT

(11) **EP 0 742 695 B1**
(45) Veröffentlichungstag und Bekanntmachung des Hinweises auf die Patenterteilung: **16.09.1998**
(21) Anmeldenummer: 95905023.8
(22) Anmeldetag: 30.12.1994
(51) Int. Cl.: A61B 17/28

(54) **MIKROCHIRURGISCHES INSTRUMENT**
MICROSURGERY INSTRUMENT
INSTRUMENT MICROCHIRURGICAL

(30) Priorität: 05.01.1994 DE 4400409
(43) Veröffentlichungstag der Anmeldung: 20.11.1996
(73) Patentinhaber: Kleihues, Hein, 10623 Berlin (DE)
(72) Erfinder: Kleihues, Hein, 10623 Berlin (DE)
(74) Vertreter: Münich, Wilhelm, Dr.
(86) Internationale Anmeldenummer: DE9401553
(87) Internationale Veröffentlichungsnummer: WO9518573

(56) Entgegenhaltungen:
- EP-A- 0 150 245
- DE-U- 9 303 366
- FR-A- 2 469 912
- US-A- 4 657 018
- US-A- 5 217 460
- US-A- 5 254 129

## Beschreibung

Die Erfindung betrifft ein mikrochirurgisches Instrument der im Oberbegriff des Anspruchs 1 angegebenen Art.

In der Chirurgie gewinnen minimal invasive (mikorchirurgische) Operationsmethoden und die zu deren Ausführung geeigneten Instrumente immer mehr an Bedeutung. Bewährt ist deren Anwendung vor allem auch in der Arthroskopie; sie bürgert sich aber auch auf anderen Gebieten - etwa in der Abdominalchirurgie - immer mehr ein.

So ist es bekannt, bei Meniskusverletzungen, bei denen eine Entfernung des Meniskus unumgänglich ist, diese Entfernung mit Hilfe von Instrumenten vorzunehmen, die durch eine enge transkutane Öffnung im Bereich des Kniegelenks bis zum verletzten Meniskus bzw. zur Kapselhinterwand vorgeschoben werden.

In einem ersten - in sich bereits mehrschrittigen - Arbeitsgang wird mittels einer Stanzzange bzw. Hakenstanze, wie sie etwa in der Druckschrift DE 38 02 907 A1 (in einer speziellen Ausbildung mit Absaugvorrichtung) beschrieben ist, der Meniskus entlang seiner Basis durch aneinander anschließende Stanzungen perforiert. Kurz bevor der Meniskus auf diese Weise vollständig von der Kapselhinterwand abgetrennt ist, wird dieser Arbeitsgang beendet. Nun wird an Stelle der Stanzzange das distale Ende einer Faßzange eingeführt, wie sie etwa (in einer speziellen Ausbildung mit zusätzlichem Endoskop) in DE 37 38 692 A1 beschrieben ist, und mit dieser der Meniskus erfaßt, von der Kapselhinterwand abgerissen und aus dem Kniegelenk entnommen. Abschließend wird in einem dritten Arbeitsgang mit einem weiteren Instrument die Kapselhinterwand im Breich der im ersten Arbeitsgang vorgenommenen Perforationen geglättet.

Dieses geschilderte Vorgehen ist zeitaufwendig und birgt durch die Notwendigkeit, nacheinander mehrere Instrumente in exakter Abstimmung auf den jeweils vorhergehenden Arbeitsschritt intrakorporal zu positionieren, gewisse Fehlerquellen und somit potentielle Risiken für den Patienten. Zudem ist die Bereitstellung, Sterilisierung und Handhabung mehrerer verschiedener Instrumente materialaufwendig und umständlich.

Auch bei Blinddarm- und Gallenoperationen wird zunehmend endoskopisch operiert, wobei die Notwendigkeit der Unterbindung und Durchtrennung von Gefäßen besteht.

Hierbei werden in einem ersten und zweiten Arbeitsschritt zunächst mittels einer speziellen Zange - einer sogenannten Ligaturzange - Gefäßverschlußelemente (Ligaturclips) beidseitig einer vorgesehenen Trennstelle am Gefäß angebracht, mit denen dieses dauerhaft verschlossene wird. Anschließend wird in einem dritten Arbeitsgang mittels einer geeigneten Vorrichtung - etwa einer mikrochirurgischen Schere nach DE 38 08 877 A1 - des Gefäß zwischen den Ligaturclips durchtrennt.

Auch dieses Vorgehen, das wiederum mehrere Arbeitsschritte und zwei verschiedene Instrumente erfordert, hat die oben erwähnten Nachteile.

US-5.127.460 beschreibt ein mikrochirurgisches Instrument mit einer an dessen distalem Ende vorgesehenen Greifzange aus einem mit einem Hohlschaft feststehend verbundenen ersten Maulteil und einem an dem Hohlschaft schwenkbar angeordneten zweiten Maulteil, das über eine Schubstange, die durch den Hohlschaft verläuft, durch ein am entgegengesetzten Ende vorgesehenen Griffteil betätigt werden kann. Zusätzlich weist dieses Instrument im Hohlschaft weitere Hohlkanäle auf, durch die ein über eine Glasfaser geleiteter Laser sowie weitere Katheter für Saug- oder Ätzvorrichtungen oder für sonstige zur Gewebeentfernung durch Lasereinwirkung benötigten Vorrichtungen bis zum distalen Ende des Hohlschaftes durchgeschoben werden können und dann aus den einander zugewandten Maulteilseiten oder aus beiden Maulteilenden herausragen.

Solch ein laserbetriebenes mikrochirurgisches Instrument hat den Vorteil, daß die für die Laserbehandlung weiterhin benötigten Instrumente in einer einzigen Sonde integriert sind, wodurch sich das zwischenzeitliche Ein- und Ausführen von Instrumenten aus dem Stichkanal erübrigt.

Nachteilig daran ist jedoch die ausschließlich auf die Gewebeentfernung durch Laserstrahlung beschränkte Verwendungsmöglichkeit. Da der Laser entweder zwischen oder vor den Maulteilen an festgelegten Positionen herausragt, kann nur innerhalb oder vor der Greifzange befindliches Gewebe durchtrennt werden. Insbesondere ist es mit diesem Instrument nicht möglich, Gewebetrennungen seitlich der Greifzange durchzuführen, was etwa bei der Meniskusentfernung im Innern einer konkaven Kapselwand von Vorteil wäre.

Der Erfindung liegt daher die Aufgabe zugrunde, ein mikrochirurgisches und insbesondere mechanisch arbeitendes Instrument bereitzustellen, das bei den geschilderten und ähnlichen Operationen eine Verkürzung der Operationszeit ermöglicht und gleichzeitig eine Vereinfachung und Verringerung der Anzahl potentieller Fehlerquellen erbringt und insbesondere eingeklemmtes Gewebe dicht seitlich des eingeklemmten Bereiches abtrennt.

Diese Aufgabe wird durch ein mikrochirurgisches Instrument mit den Merkmalen nach Anspruch 1 gelöst.

Die Erfindung schließt den Gedanken ein, ein mikrochirurgisches Instrument zu schaffen, das die Ausführung der Funktionen des Erfassens bzw. Einklemmens (eines Gewebsteiles, Gefäßes o.ä.) und des Erzeugens eines Schnittes in diesem, insbesondere seiner Durchtrennung, mit ein und demselben Instrument und daher in einem Arbeitsgang ermöglicht, und das die entsprechenden Funktionselemente in sich vereinigt.

Sie geht dabei von bekannten endoskopisch einsetzbaren bzw. mikrochirurgischen Instrumenten aus, bei denen die Funktion des Erfassens, Einklemmens oder Stanzens durch zwei über geeignete Griffelemente gegeneinander verschwenkbare Zangen- bzw. Stanzzangenteile erfüllt wird, und fügt diesem Funktionsprinzip das eines translatorischen Schneidvorganges hinzu, der mittels eines weiteren Griffelementes gesteuert wird. Zwischen beiden Vorgängen besteht ein enger Funktionszusammenhang insofern, als beim Schneiden in vorteilhafter Weise das Erfaßt- bzw. Eingeklemmtsein des zu schneidenden Gewebsteils bzw. Gefäßes und zugleich die durch die Zangenteile gelieferte Positionierungs-Vorgabe für die Schneidvorrichtung genutzt wird.

Neben dem Vorteil, daß die bislang aufeinanderfolgenden und das Einführen getrennter Instrumente erfordernden Arbeitsschritte des Erfassens bzw. Einklemmens und des Schneidens nunmehr nach einem einzigen Einführen ein und desselben Instrumentes nahezu gleichzeitig ausgeführt werden können, wird damit auch die Gefahr von Positionierungsfehlern oder eines "Verlorengehens" eines abgetrennten Gewebsteiles (etwa eines abgetrennten Meniskus im Kniegelenk) entscheidend verringert.

Die Schneidvorrichtung weist in einer vorteilhaften Ausbildung eine zwischen zwei gegenüber dem distalen Ende des Außenschaftes seitlich der Maulteile der Greif- bzw. Klemmvorrichtung axial ausfahrbaren Führungsstäben oder -drähten gelagerte, im wesentlichen gleichgerichtet mit diesen verschiebbare Schneidklinge auf.

Insbesondere für den Einsatz des Instruments in der Arthroskopie - speziell etwa für die Abtrennung eines Meniskus von der konkav gekrümmten hinteren Kniegelenkkapselwand - ist es von Vorteil, wenn die Führungsstäbe oder -drähte im teilweise und ganz ausgefahrenen Zustand senkrecht zu ihrer Längsachse flexibel, insbesondere in Richtung auf die Maulteile hin biegbar, ausgebildet sind. Sie können dann nämlich entlang einer gekrümmten Auflagefläche eine gekrümmte Bahn beschreiben und damit einen ebensolchen Schnitt ermöglichen.

Auch das Instrument als Ganzes kann eine gebogene Form aufweisen, um die Handhabung in Körperhöhlen, Gelenken usw. zu erleichtern.

Die Schneidklinge kann mit den Führungsstäben fest verbunden, grundsätzlich aber auch diesen gegenüber verschiebbar sein. Ersteres ist konstruktiv einfacher, letzteres ermöglicht es, zunächst durch das Ausfahren der Führungen die Schnittebene festzulegen, zu prüfen und ggf. noch zu korrigieren und erst danach den Schnitt auszuführen.

Die Schneidvorrichtung kann eine rein mechanisch wirkende Klinge - etwa aus Edelstahl oder einem anderen Metall und ggf. mit einer Gleitbeschichtung - oder auch ein Elektrokautermesser aufweisen. Im letzteren Falle wird gleichzeitig mit dem Schnitt in günstiger Weise eine Gewebskoagulation an den Schnittflächen erreicht.

Zur Vermeidung unerwünschter Gewebs- oder Gefäßperforationen ist es - insbesondere, wenn die Schneidvorrichtung an gekrümmten Auflageflächen (Gelenkkapseln o.ä.) eingesetzt werden soll - günstig, wenn die Führungsstäbe oder -drähte jeweils eine distal angeordnete, verrundete Schutzkappe aufweisen. Deren Form kann bei einer speziellen Gestaltung des Instrumenets für einen bestimmten Einsatzzweck - etwa für Meniskusabtrennungen - auf diesen abgestimmt sein.

Es erleichtert das Einführen des Instruments in den Körper und vermeidet Verletzungen während des Einführens, wenn die Zangen- bzw. Maulteile jeweils eine mit der Gestalt der Schutzkappen korrespondierende Ausnehmung aufweisen, in der die Schutzkappen im eingefahrenen Zustand der Schneidvorrichtung und bei geschlossenen Zangenteilen derart aufgenommen sind, daß das distale Ende des Instruments zum Einführen in den Körper eine geschlossene, konvex gewölbte Oberfläche aufweist. Zusätzlich kann es zweckmäßig sein, daß die Schutzkappen und/oder die Ausnehmungen eine Arretierungsvorrichtung aufweisen, die die Schutzkappen im eingefahrenen Zustand mit den Zangenteilen arretiert. Diese können dann auch bei Widerstanden während des Einführens nicht aus den Ausnehmungen gedrückt werden.

Eine besonders kostensparende Ausgestaltung besteht darin, daß die Schneidvorrichtung vom übrigen Instrument lösbar und austauschbar ausgebildet ist. Sie kann dann zum einen in gleicher, standardisierter Form für verschieden gestaltete Instrumente gefertigt und zum anderen nach Abnutzung als (preisgünstiges) Einzelteil ausgetauscht werden.

Ein vorteilhafte konstruktive Ausbildung des Instruments, die ebenfalls Baukastenlösungen ermöglicht, besteht darin, daß der Außenschaft mindestens in einem distalen Abschnitt seiner Längserstreckung zweiteilig ausgeführt ist, wobei im ersten Teil die erste Schubstange und im zweiten Teil die zweite Schubstange und die Schneidvorrichtung aufgenommen sind, und daß insbesondere mindestens ein distaler Abschnitt des zweiten Teils des Außenschaftes zusammen mit der Schneidvorrichtung vom übrigen Instrument lösbar und austauschbar ausgebildet ist.

Die erfindungsgemäße Lösung kann breite Anwendung in vielen Feldern der Mikrochirurgie finden. Insbesondere kann sie eine Clipzange zum Anbringen von Ligaturclips zum dauerhaften Gefäßverschluß und gleichzeitig zum Durchtrennen des verschlossenen Gefäßes oder eine Meniskus-Faßzange mit integriertem Meniskotom darstellen.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 eine seitliche Gesamtansicht einer Ausführungsform des erfindungsgemäßen Instruments,
Figur 2 eine schematische Querschnittsdarstellung der in Fig. 1 gezeigten Ausführungsform in der Ebene A-A',
Figur 3 eine perspektivische Darstellung des distalen Bereiches B der in Fig. 1 gezeigten Ausführungsform,
Figur 4a bis 4e eine Detaildarstellung einer Ausführungsform der Schneidvorrichtung des erfindungsgemäßen Instruments und
Figur 5a bis 5f Detaildarstellungen von gegenüber Fig. 1 abgewandelten Ausführungsformen der Erfindung.

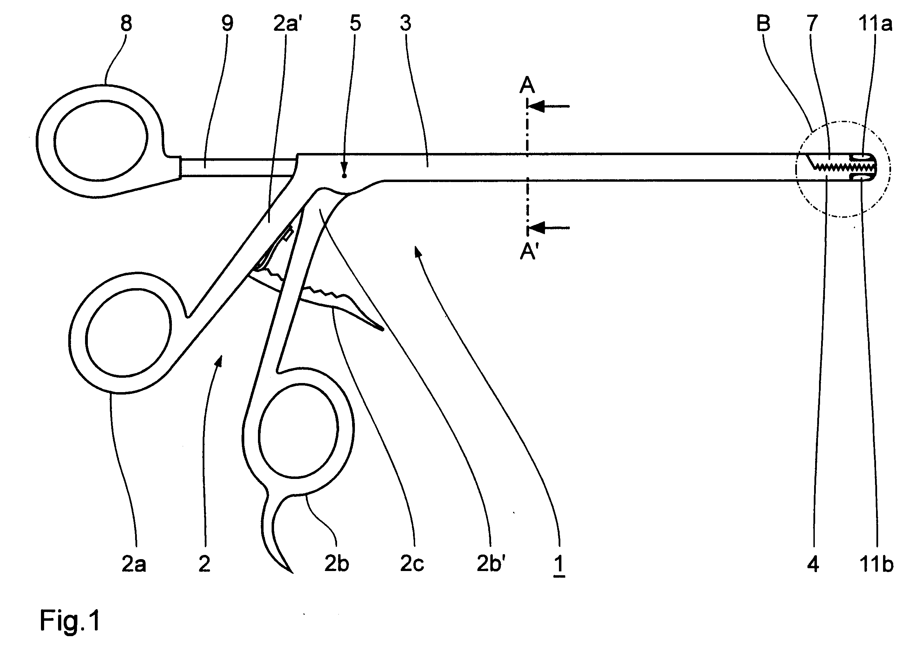

In Fig. 1 ist eine Ausführungsform des erfindungsgemäßen Instruments als Meniskus-Faßzange mit integriertem Meniskotom 1 in einer Seitenansicht dargestellt.

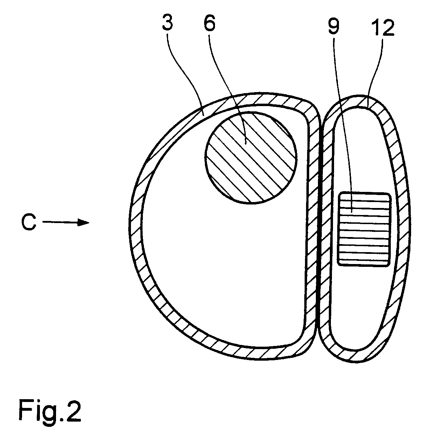

Die Meniskus-Faßzange 1 weist einen Scherengriff 2 mit zwei Griffstücken 2a und 2b sowie einem Feststellriegel 2c zur Betätigung auf. Das erste Scherengriffstück 2a mündet an seinem vorderen Ende 2a' in einen annähernd hohlzylindrischen Schaft 3, der an seinem distalen Ende in ein erstes Maulteil 4 ausläuft. Das zweite Scherengriffstück 2b ist an seinem vorderen Ende 2b' distal von einer im Schaft 3 gelagerten Drehachse 5 mit einer (in Fig. 1 nicht dargestellten, aber in Fig. 2 zu erkennenden) ersten Schubstange 6 verbunden, die an ihrem distalen Ende ein zweites Maulteil 7 trägt, das eine zweite (nicht gezeigte) Drehachse aufweist. Das zweite Maulteil 7 ist durch eine Bewagung des zweiten Griffstücks 2b relativ zum ersten Griffstück 2a, die durch die beiden Drehachsen und die Schubstange 6 umgesetzt und auf das zweite Maulteil 7 übertragen wird, gegenüber dem ersten Maulteil 4 verschwenkbar sowie über den Feststellriegel 2c in der eingenommenenen Stellung arretierbar.

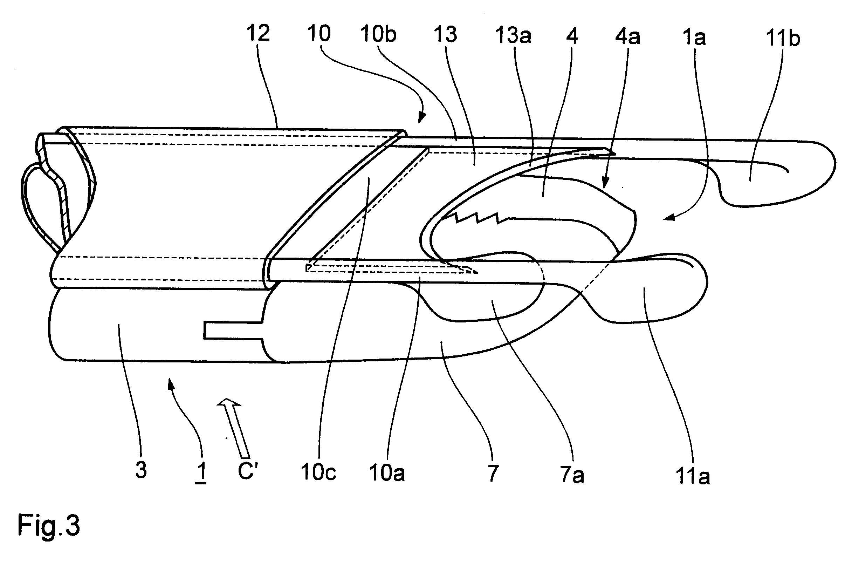

Neben dem Scherengriff 2 mit seinen beiden Griffstücken 2a, 2b weist die Meniskus-Faßzange nach Fig. 1 ein weiteres, drittes Griffstück 8 auf, das das proximale Ende einer zweiten Schubstange 9 bildet. An derem distalen Ende ist eine Schneidvorrichtung 10 angebracht, die in Fig. 3 genauer gezeigt ist und von der in Fig. 1 nur Endnocken der Verdickungen bzw. Schutzkappen 11a und 11b zu erkennen sind.

Fig. 2 zeigt eine schematische Querschnittsdarstellung der Schaftanordnung der in Fig. 1 dargestellten Meniskus-Faßzange 1 in der in Fig. 1 gekennzeichneten Schnittsbene A-A'. Die in Fig. 1 zugrundegelegte Blickrichtung ist in Fig. 2 durch den Pfeil C bezeichnet.

In der Figur ist die im (ersten) Hohlschaft 3 angeordnete erste Schubstange 6 zur Betätigung des zweiten Maulteils zu erkennen. Weiter ist zu sehen, daß die zweite Schubstange 9 zur Betätigung der Schneidvorrichtung in einem gesonderten, mit dem Hohlschaft 3 unbeweglich verbundenden, zweiten Hohlschaft 12 angeordnet ist.

Fig. 3 zeigt eine perspektivische Darstellung des in Fig. 1 mit "b" gekennzeichneten distalen Abschnitts der Meniskus-Faßzange 1, von deren Oberkante her schräg auf die Rückseite gesehen, mit halb ausgefahrener Schneidvorrichtung 10. (Die in Fig. 1 zugrundegelegte Blickrichtung ist in Fig. 3 durch den Pfeil C' verdeutlicht.)

Auf der linken Seite von Fig. 3 ist nochmals die Anordnung der beidenn Hohlschäfte 3 und 12 gezeigt, wobei an dieser Stelle im Hohlschaft 12 nicht mehr die zweite Schubstange 9, sondern bereits die distal mit dieser verbundene Schneidvorrichtung 10 im Querschnitt zu sehen ist.

Die im inneren des zweiten Hohlschaftes 12 verschiebliche, lösbar mit der zweiten Schubstange 9 verbundene und somit austauschbare Schneidvorichtung 10 weist zwei seitliche, biegsame Kunststoff-Führungsstäbe 10a und 10b und einem diese verbindenden Steg 10c auf. Die Führungsstäbe 10a und 10b laufen distal in jeweils eine nockenähnliche Verdickung bzw. Schutzkappe 11a und 11b aus. Das erste und zweite Maulteil 4 und 7 weisen in ihrem distalen, zu einer gemeinsamen, konvex gekrümmten Stirnfläche 1a der Faßzange 1 verrundeten Bereich jeweils eine der Form der Verdickungen 11a und 11b angepaßte Ausnehmung 4a bzw. 7a auf, in der die Verdickungen im vollständig eingezogenenen Zustand der Schneidvorrichtung 10 ruhen.

Distal vom Steg 10c, in diesen fortsetzender Anordnung, ist eine Edelstahl-Schneidklinge 13 mit zwischen den Führungsstäben 10a und 10b angeschrägter und gleichzeitig konkav gekrümmter Vorderkante 13a befestigt. Die Vorderkante 13a hat einen Abstand von einigen Millimetern zu den Verdickungen 11a und 11b und ist zu den Maulteilen 4 und 7 hin angeschrägt bzw. angeschliffen.

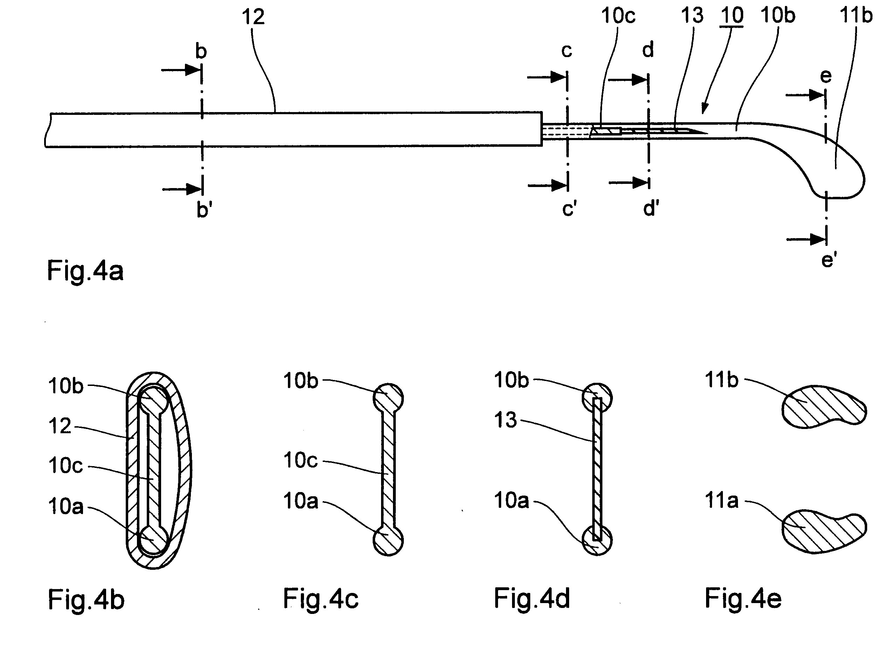

Fig. 4a bis 4e zeigen als Detaildarstellung in einer Seitenansicht und vier Querschnittsdarstellungen nochmals genau den Aufbau der Schneidvorrichtung 10 im zweiten Hohlschaft 12. Die Figuren 4b bis 4e sind Querschnittdarstellungen in den durch entsprechend mit b, b', c, c', d, d' und e, e' bezeichnete Pfeile gekennzeichneten Schnittebenen der Seitenansicht nach Fig. 4a.

Die Teile und zugehörigen Bezugsziffern sind dieselben wie in Fig. 3, so daß die Beschreibung insoweit hier nicht wiederholt wird.

Nachfolgend wird die Funktionsweise der in den Figuren 1 bis 3 gezeigten Meniskus-Faßzange mit integriertem Mikrotom beschrieben.

Zunächst wird das Instrument mit geschlossenen Maulteilen 4 und 7 und voll in die Ausnehmungen 4a und 7a versenkten Verdickungen bzw. Schutzkappen 11a und 11b in üblicher Weise durch einen transkutanen Zugang im Bereich des Kniegelenks positioniert. Dann werden zunächst durch Betätigung des Scherengriffes 2 die Maulteile 4 und 7 auseinandergeschwenkt, um den zu entfernenden Meniskus geführt und dieser durch Zusammenklappen der Maulteile erfaßt.

Dann wird mittels des Griffstücks 8 über die Schubstange 9 die Schneidvorrichtung 10 vorgeschoben, wobei die Führungsstäbe 10a und 10b um die Meniskusbasis herum an der konkav gekrümmten hinteren Kapselwand entlanggleiten und eine ebenso gekrümmte Schnittebene für die Schneidklinge 13 festlegen. Die den distalen Verdickungen bzw. Schutzkappen 11a und 11b im konstruktiv festgelegten Abstand folgende, in Längsrichtung hinreichend flexible Schneidklinge trennt - der vorgegebenen Schnittebene folgend - den Meniskus an dessen Basis von der Kapselwand ab, wobei er zwischen den Maulteilen 4 und 7 erfaßt bleibt und nach dem Abtrennen mit dem Instrument zusammen entnommen werden kann.

Die beispielsweise nockenartigen Verdickungen bzw. Schutzkappen 11a und 11b ermöglichen zusammen mit der Biegsamkeit der Schneidvorrichtung ein relativ leichtes Gleiten der Führungsstäbe 10a und 10b an der Kapselwand, ohne daß die Gefahr eines Eindringens in diese und damit eines Verhakens der Schneidvorrichtung besteht. Beim Trennvorgang ermöglicht die abgeschrägte Gestaltung der Schneidklinge in vorteilhafter Weise einen wenig kraftaufwendigen und präzisen Schnitt unter Vermeidung von Gewebszusammenballungen an einem der Führungsstäbe. Eine Gleitbeschichtung der Schneidklinge erleichtert das Schneiden zusätzlich. Die Abtrennung kann durch Ziehen an dem während des Schneidens mit dem Maulteilen erfaßten Meniskus zusätzlich befördert werden.

Das beschriebene Vorgehen erfordert einen weit geringeren Zeit- und instrumentellen Aufwand und ist mit geringeren Risiken behaftet als das herkömmliche operative Vorgehen.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht.

Insbesondere sind vielgestaltige Abwandlungen der Form des gesamten Instruments - das etwa in Anpassung an spezielle Einsatzzwecke in Längs- und/oder Querrichtung gebogen sein kann - wie auch der Betätigungselemente - die etwa auch Zangengriffen bzw. T-förmigen Druckstücken nachempfunden sein können -, der Maulteile und der Schneidvorrichtung im einzelnen möglich.

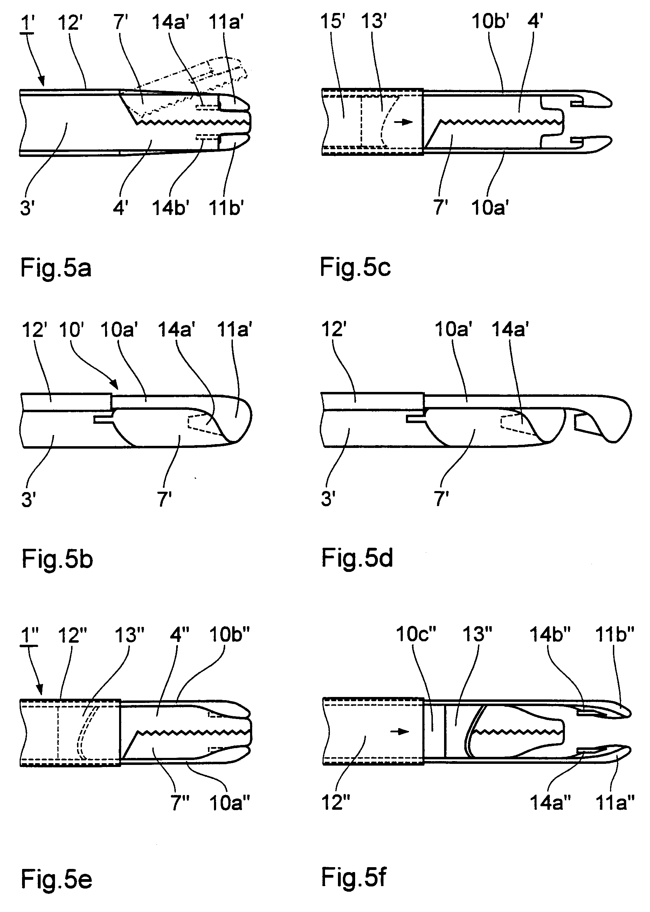

Zwei derart abgewandelte Ausführungsformen sind in den Figuren 5a bis 5d bzw. 5e und 5f skizziert.

Die in Fig. 5a entsprechend der Blickrichtung von Fig. 1, in Fig. 5c von der entgegengesetzten Seite und in Fig. 5b und 5d von der Oberkante der Faßzange aus gezeigte Ausführungsform weist dieselben Hauptbestandteile auf wie die nach Fig. 1 bis 3. Diese sind mit den entsprechenden, einfach gestrichenen, Bezugsziffern bezeichnet und werden nachfolgend nicht nochmals beschrieben. Fig. 5a und 5b zwigen das Instrumnet mit eingezogenere und Fig. 5c und 5d mit teilweise ausgefahrener Schneidvorrichtung.

Das Instrument unterscheidet sich von dem nach Fig. 1 bis 3 durch die veränderte Form der Maulteile 4' und 7' und der Nocken bzw. Schutzkappen 11a' und 11b', die hier nach proximal gerichtete Arretierungsstifte 14a' bzw. 14b' aufweisen, die im eingezogenen Zustand der Schneidvorrichtung 10' in entsprechenden Ausnehmungen in den Maulteilen 4' und 7' ruhen und die Führungsstäbe 10a' und 10b' seitlich allseitig arretieren.
Es unterscheidet sich von der Faßzange nach Fig. 1 bis 3 weiterhin dadurch, daß - wie in Fig. 5c angedeutet - die Schneidklinge 13' nicht fest mit den Führungsstäben 10a' und 10b' verbunden, sondern zwischen diesen verschieblich geführt ist und über ein gesondertes Druckstück 15' vorgeschoben werden kann, nachdem zuerst die Führungsstäbe ausgefahren wurden.

Fig. 5e und 5f zeigen in einer entsprechenden Detaildarstellung eine weitere Ausführungsform aus zur Blickrichtung in Fig. 1 entgegengesetzter Blickrichtung mit eingezogener (Fig. 5e) bzw. teilweise ausgefahrener Schneidvorrichtung (Fig. 5f). Auch hier entsprechen die Hauptteile denen der Fig. 1 bis 3, sind entsprechend (zweigestrichen) bezeichnet und werden nicht nochmals erläutert. Hier sind an die Stelle der Kunststoff-Führungsstäbe gewendelte Federstahl-Führungsdrähte 10a'' und 10b'' getreten, die Schneidklinge 13'' ist mit diesen verschweißt, und auf das vordere Ende sind Kunststoffkappen 11a'' bzw. 11b'' mit Arretierungsnoppen 14a'' bzw. 14b'' aufgepreßt. Deren Form entsprechend, sind wieder Ausnehmungen in den Maulteilen 4' bzw. 7' vorgesehen. Bei dieser Ausführung ist - was in den Detaildarstellungen nicht gezeigt ist - die Schneidvorrichtung 10'' einschließlich des sie aufnehmenden und führenden Hohlschaftes 12'' mit dem übrigen Instrument durch eine Klemm- oder Schraubverbindung verbunden und austauschbar.

Bei den Ausführung der Erfindung als Meniskus-Faßzange oder allgemeiner für arthroskopische Anwendungen kann es zweckmäßg sein, gesondert links- und rechtschneidende Ausführungen zu fertigen, die eine bezüglich der Vertikalebene des Instruments zueinander spiegelsymmetrische Anordnung und Ausbildung der Schneidvorrichtung haben.

Ausführungen für den Gefäßverschluß und die Gefäßdurchtrennung in der Abdominalchirurgie weisen vorzugsweise ein zum Setzen von Ligaturclips ausgebildetes - als solches bekanntes - Zangenteil sowie eine gegenüber dem Meniskotom abgewandelt ausgebildete Schneidvorrichtung auf. Da hier die Führungsstäbe bzw. -drähte nicht an einer gekrümmten Fläche entlang, sondern etwa um die Außenwandung eines Gefäßes gleiten müssen, werden insbesondere die Form der Verdickungen und ggf. auch die Biegeeigenschaften der Führungsstäbe von der bei den oben beschriebenen Beispielen abweichen.

Als Schneidvorrichtung kann auch ein entsprechend angepaßtes Elektrokautermesser vorgesehen sein.

Die erläuterten Ausführungsformen sind auch hinsichtlich der Materialauswahl nur als zweckmäßige Beispiele zu verstehen; es kann im übrigen auf in der Medizintechnik für vergleicbare Anwendungen bewährte Materialien zurückgegriffen werden.

## Patentansprüche

1. Mikrochirurgisches Instrument (1; 1'; 1''), insbesondere für die operative Endoskopie/Arthroskopie, mit einem aus dem distalen Ende eines an seinem anderen Ende mit einem ersten Griffteil (2a) verbundenen Hohlschaftes (3; 3') hervorstehenden, am Hohlschaft feststehenden ersten Maulteil (4; 4'; 4'') sowie einem an diesem schwenkbar angeordneten zweiten Maulteil (7; 7'; 7''), das mittels einer durch den Hohlschaft verlaufenden ersten Schubstange (6) über ein an deren anderem Ende vorgesehenes zweites Griffteil (2b) betätigt werden kann, um einen Erfassungs- oder Einklemmvorgang auszuführen,
**dadurch gekennzeichnet,** daß
am distalen Ende des Hohlschaftes (3; 3') eine mittels einer in diesem axial verschiebbaren, zweiten Schubstange (9) über ein drittes Griffteil (8) betätigbare Schneidvorrichtung (10; 10'; 10'') vorgesehen ist, um zusätzlich zum Erfassungs- oder Einklemmvorgang einen Schneidvorgang am erfaßten Gewebsabschnitt auszuführen.

2. Mikrochirurgisches Instrument nah Anspruch 1 , **dadurch gekennzeichnet,** daß die Schneidvorrichtung (10; 10'; 10'') eine zwischen zwei gegenüber dem distalen Ende des Hohlschaftes (3; 3') seitlich der Maulteile (4, 7; 4', 7'; 4'', 7'') axial ausfahrbaren Führungsstäben oder -drähten (10a, 10b; 10a', 10b'; 10a'', 10n'') gelagerte, im wesentlichen gleichgerichtet mit diesen verschiebbare Schneidklinge (13; 13'; 13'') aufweist.

3. Mikrochirurgisches Instrument nach Anspruch 2, **dadurch gekennzeichnet,** daß die Führungsstäbe oder -drähte (10a, 10b; 10a', 10b'; 10a'', 10b'') im teilweise und ganz ausgefahrenen Zustand senkrecht zu ihrer Längsachse flexibel, insbesondere in Richtung auf die Maulteile hin biegbar, ausgebildet sind.

4. Mikrochirurgisches Instrument nach Anspruch 2 oder 3, **dadurch gekennzeichnet,** daß die Schneidklinge (13; 13'') mit den Führungsstäben oder -drähten (10a, 10b; 10a'', 10b'') fest verbunden ist, wobei ihre Vorderkante (13a) zu den distalen Enden der Führungsstäbe oder -drähte einen Abstand aufweist.

5. Mikrochirurgisches Instrument nach einem der Ansprüche 2 bis 4 , **dadurch gekennzeichnet,** daß die Schneidklinge (13; 13'; 13''), vorzugsweise in Richtung auf die Maulteile (4, 7; 4', 7'; 4'', 7'') hin, angeschliffen ist.

6. Mikrochirurgisches Instrument nach einem der Ansprüche 2 bis 5, **dadurch gekennzeichnet,** daß die Schneidklinge (13; 13'; 13'') eine vorzugsweise konkav gekrümmte Vorderkante (13a) aufweist.

7. Mikrochirurgisches Instrument nach Anspruch 1, **dadurch gekennzeichnet,** daß die Schneidvorrichtung ein Elektrokautermesser aufweist.

8. Mikrochirurgisches Instrument nach einem der Ansprüche 2 bis 7, **dadurch gekennzeichnet,** daß die Führungsstäbe oder -drähte (10a, 10b; 10a', 10b'; 10a'', 10b'') jeweils eine distal angeordnete, verrundete Verdickung oder Schutzkappe (11a, 11b; 11a', 1b'; 11a'', 11b'') aufweisen.

9. Mikrochirurgisches Instrument nach Anspruch 8 , **dadurch gekennzeichnet,** daß das erste und zweite Maulteil (4, 7; 4', 7'; 4'', 7'') jeweils eine mit der Gestalt der Verdickungen bzw. Schutzkappen (11a, 1b; 11a', 11b'; 11a'', 11b'') korrespondierende Ausnehmung aufweisen, in der die Verdickungen bzw. Schutzkappen im eingefahrenen Zustand der Schneidvorrichtung (10; 10'; 10'') und bei geschlossenen Maulteilen derart aufgenommen sind, daß das distale Ende des Instruments (1; 1'; 1'') zum Einführen in den Körper eine geschlossene, konvex gewölbte Oberfläche (1a) aufweist.

10. Mikrochirurgisches Instrument nach Anspruch 9 , **dadurch gekennzeichnet,** daß die Schutzkappen (11a', 11b'; 11a'', 11b'') und/oder die Ausnehmungen eine Arretierungsvorrichtung (14a', 14b'; 14a'', 14b'') aufweisen, die die Schutzkappen im eingefahrenen Zustand mit den Maulteilen (4', 7'; 4'', 7'') arretiert.

11. Mikrochirurgisches Instrument nach einem der vorangehenden Ansprüche, **dadurch gekennzeichnet,** daß die Schneidvorrichtung (10; 10'; 10'') vom übrigen Instrument lösbar und austauschbar ausgebildet ist.

12. Mikrochirurgisches Instrument nach einem der vorangehenden Ansprüche, **dadurch gekennzeichnet,** daß der Hohlschaft mindestens in einem distalen Abschnitt seiner Längserstreckung zweiteilig ausgeführt ist, wobei im ersten Teil (3; 3'; 3'') die erste Schubstange (6) und im zweiten Teil (12; 12'; 12'') die zweite Schubstange (9) und die Schneidvorrichtung (10; 10'; 10'') aufgenommen sind.

13. Mikrochirurgisches Instrument nach Anspruch 12 , **dadurch gekennzeichnet,** daß mindestens ein distaler Abschnitt des zweiten Teils des Hohlschaftes zusammen mit der Schneidvorrichtung vom übrigen Instrument lösbar und austauschbar ausgebildet ist.

14. Mikrochirurgisches Instrument nach einem der vorangehenden Ansprüche, **dadurch gekennzeichnet,** daß das erste und zweite Maulteil mit den zugehörigen Betätigungselementen eine Clipzange zum Anbringen von Ligaturclips zum dauerhaften Gefäßverschluß bilden.

15. Mikrochirurgisches Instrument nach einem der Ansprüche 1 bis 13 , **dadurch gekennzeichnet,** daß das erste und zweite Maulteil (4, 7; 4', 7'; 4''; 7'') mit den zugehörigen Betätigungselementen (3, 6) eine Meniskus-Faßzange (1: 1'; 1'') bilden.

## Claims

1. Microsurgery instrument (1; 1'; 1''), particularly for endoscopic/arthroscopic surgery, including a first jaw element (4; 4'; 4'') which projects from the distal end of a hollow shaft (3; 3') connected by its other end to a first handle element (2a) and which is stationary on said hollow shaft, as well as a second jaw element (7; 7'; 7'') arranged to pivot thereon, which is adapted to be operated by means of a first connecting-rod (6) extending through said hollow shaft via a second handle element (2b) disposed on the other end of said connecting rod, for performing a seizing or clamping operation,
**characterized in** that
a cutting means (10; 10'; 10'') is provided on the distal end of said hollow shaft (3; 3'), which is operable via a third handle element (8) by means of a second connecting-rod (9) axially displaceable in said hollow shaft, for performing a cutting operation on the seized tissue section in addition to said seizing or clamping operation.

2. Microsurgery instrument according to Claim 1, **characterized in** that said cutting means (10; 10'; 10'') includes a cutting blade (13; 13'; 13'') supported between two axially extensible guiding rods or wires (10a, 10b; 10a', 10b'; 10a'', 10b'') laterally of said jaw elements (4, 7; 4', 7'; 4'', 7'') opposite to the distal end of said hollow shaft (3; 3'), which blade is displaceable in substantially the same direction together with said guiding rods or wires.

3. Microsurgery instrument according to Claim 2, **characterized in** that said guiding rods or wires (10a, 10b; 10a', 10b'; 10a'', 10b'') are configured to be flexible, particularly pliable in a direction towards said jaw elements, in a direction normal on their longitudinal axis in the partly or completely extended state.

4. Microsurgery instrument according to Claim 2 or 3, **characterized in** that said cutting blade (13; 13'') is fixedly connected to said guiding rods or wires (10a, 10b; 10a'', 10b''), with its leading edge (13a) being spaced from the distal ends of said guiding rods or wires.

5. Microsurgery instrument according to any of the Claims 2 to 4, **characterized in** that said cutting blade (13; 13'; 13'') is partially ground, preferably in a direction towards said jaw elements (4, 7; 4', 7'; 4'', 7'').

6. Microsurgery instrument according to any of the Claims 2 to 5, **characterized in** that said cutting blade (13; 13'; 13'') presents a leading edge (13a) preferably provided with a concave bend.

7. Microsurgery instrument according to Claim 1, **characterized in** that said cutting means includes a galvano-cautery blade.

8. Microsurgery instrument according to any of the Claims 2 to 7, **characterized in** that each of said guiding rods or wires (10a, 10b; 10a', 10b'; 10a'', 10b'') includes a radiused thickening or protective cap (11a, 11b; 11a', 11b'; 11a'', 11b''), respectively.

9. Microsurgery instrument according to Claim 8, **characterized in** that each of said first and second jaw elements (4, 7; 4', 7'; 4'', 7'') includes a recess in correspondence with the shape of said thickened portions or protective caps (11a, 11b; 11a', 11b'; 11a'', 11b''), in which said thickened portions or protective caps, respectively, are received when said cutting means (10; 10'; 10'') is retracted and said jaw elements are closed, such that the distal end of the instrument (1; 1'; 1'') presents a continuous surface (1a) having a convex curvature for introduction into the body.

10. Microsurgery instrument according to Claim 9, **characterized in** that said protective caps (11a', 11b'; 11a'', 11b'') and/or said recesses include a locking means (14a', 14b'; 14a'', 14b'') which locks said protective caps in the retracted state with said jaw elements (4', 7'; 4'', 7'').

11. Microsurgery instrument according to any of the preceding Claims, **characterized in** that said cutting means (10; 10'; 10'') is configured to be detachable from the remaining parts of the instrument and to be exchangeable.

12. Microsurgery instrument according to any of the preceding Claims, **characterized in** that said hollow shaft is formed in a two-part configuration at least in a distal section of its longitudinal extension, with said first connecting-rod (6) being received in the first part (3; 3'; 3'') and with said second connecting rod (9) and said cutting means (10; 10'; 10'') being received in the second part (12; 12'; 12'').

13. Microsurgery instrument according to Claim 12, **characterized in** that at least a distal section of said second part of said hollow shaft, together with said cutting means, is configured to be detachable from the remaining parts of the instrument and to be exchangeable.

14. Microsurgery instrument according to any of the preceding Claims, **characterized in** that said first and second jaw elements with the appertaining operating elements form clipping forceps for applying ligating clips for permanent vascular occlusion.

15. Microsurgery instrument according to any of the Claims 1 to 13, **characterized in** that said first and second jaw elements (4, 7; 4', 7'; 4'', 7'') with the appertaining operating elements (3, 6) form meniscus-holding forceps.

## Revendications

1. Instrument microchirurgical (1; 1'; 1''), en particulier pour l'endoscopie/arthroscopie chirurgicale, comprenant un premier élément de mâchoire (4; 4'; 4'') en saillie de l'extrémité distale d'une tige creuse (3; 3') reliée, par son autre extrémité, à un premier élément de manche (2a), qui est immobile sur ladite tige creuse, ainsi qu'un deuxième élément de mâchoire (7; 7'; 7'') disposer pour y pivoter, qui est apte à être actionné moyennant une première belle poussante (6) qui s'étend à travers ladite tige creuse via un deuxième élément de manche (2b) disposé sur l'autre extrémité de ladite belle poussante, à effectuer un mouvement de saisie ou de serrage,
**caractérisé en ce**
qu'un moyen coupeur (10; 10'; 10'') est prévu sur l'extrémité distale de ladite tige creuse (3; 3'), qui peut être actionné va un troisième élément de manche (8) moyennant une deuxième belle poussante (9) déplaçable en sens axial dans ladite tige creuse, afin de réaliser une opération de coupe sur la partie de tissu saisie, additionnellement à ladite opération de saisie ou de serrage.

2. Instrument microchirurgical selon la revendication 1, **caractérisé en ce** que ledit moyen coupeur (10; 10'; 10'') comprend une lame (13; 13'; 13'') logé entre deux tiges ou fils de guidage (10a, 10b; 10a', 10b'; 10a'', 10b''), à sortir axialement, qui sont disposés latéralement desdits éléments de mâchoire (4, 7; 4', 7'; 4'', 7'') et opposés à l'extrémité distale de ladite tige creuse (3; 3'), cette lame étant déplaçable essentiellement en même sens, ensemble avec lesdits tiges ou fils de guidage.

3. Instrument microchirurgical selon la revendication 2, **caractérisé en ce** que lesdits tiges ou fils de guidage (10a, 10b; 10a', 10b'; 10a'', 10b'') sont conçus sous forme flexible, en particulier pliable en une direction vers lesdits éléments de mâchoire, en un sens orthogonal sur leur axe longitudinal, en état sorti partiellement ou complètement.

4. Instrument microchirurgical selon la revendication 2 or 3, **caractérisé en ce** que ladite lame (13; 13'') est solidarisée auxdits tiges ou fils de guidage (10a, 10b; 10a'', 10b''), à son arête avant (13a) étant espacée des extrémités distales desdits tiges ou fils de guidage.

5. Instrument microchirurgical selon une quelconque des revendications 2 à 4, **caractérisé en ce** que ladite lame (13; 13'; 13'') est affûtée en partie, de préférence en un sens vers lesdits éléments de mâchoire (4, 7; 4', 7'; 4'', 7'').

6. Instrument microchirurgical selon une quelconque des revendications 2 à 5, **caractérisé en ce** que ladite lame (13; 13'; 13'') présente une arête avant (13a) de préférence à une courbure concave.

7. Instrument microchirurgical selon la revendication 1, **caractérisé en ce** que ledit moyen coupeur comprend une lame galvano-cautère.

8. Instrument microchirurgical selon une quelconque des revendications 2 à 7, **caractérisé en ce** que chacun desdits tiges ou fils de guidage (10a, 10b; 10a', 10b'; 10a'', 10b'') comprend un gonflement arrondi ou respectivement un capot protecteur (11a, 11b; 11a', 11b'; 11a'', 11b'').

9. Instrument microchirurgical selon la revendication 8, **caractérisé en ce** que chacun desdits premier et deuxième éléments de mâchoire (4, 7; 4', 7'; 4'', 7'') comprend un creux en correspondance avec la forme desdites parties de gonflement ou capots protecteurs (11a, 11b; 11a', 11b'; 11a'', 11b''), dans lequel lesdits parties de gonflement ou respectivement lesdits capots protecteurs sont reçus quand ledit moyen coupeur (10; 10'; 10'') est rentré et lesdits éléments de mâchoire sont fermés, de façon que l'extrémité distale de l'instrument (1; 1'; 1'') présente une surface continue (1a) à une courbure convexe pour l'introduction dans le corps.

10. Instrument microchirurgical selon la revendication 9, **caractérisé en ce** que lesdits capots protecteurs (11a', 11b'; 11a'', 11b'') et/ou lesdits creux comprennent un moyen de verrouillage (14a', 14b'; 14a'', 14b'') à verrouiller lesdits capots protecteurs en leur état rentré avec lesdits éléments de mâchoire (4', 7'; 4'', 7'').

11. Instrument microchirurgical selon une quelconque des revendications précédentes, **caractérisé en ce** que ledit moyen coupeur (10; 10'; 10'') est conçu de façon qu'il soit détachable de la partie restante de l'instrument et échangeable.

12. Instrument microchirurgical selon une quelconque des revendications précédentes, **caractérisé en ce** que ladite tige creuse est conçue sous forme en deux pièce au moins dans une partie distale le long son extension longitudinale, à ladite première bielle poussante (6) étant reçue dans une première pièce (3; 3'; 3'') et ladite deuxième bielle poussante (9) et ledit moyen coupeur (10; 10'; 10'') étant reçus dans la deuxième pièce (12; 12'; 12'').

13. Instrument microchirurgical selon la revendication 12, **caractérisé en ce** qu'au moins une partie distale de la deuxième pièce de tige creuse, ensemble avec ledit moyen coupeur, est conçu en forme détachable de la partie restante de l'instrument et échangeable.

14. Instrument microchirurgical selon une quelconque des revendications précédentes, **caractérisé en ce** que lesdits premier et éléments de mâchoire avec les éléments d'actionnement y affectés constituent une pince de clip pour l'application des clips liants pour une ligature de vaisseaux permanente.

15. Instrument microchirurgical selon une quelconque des revendications 1 à 13 **caractérisé en ce** que lesdits premier et deuxième éléments de mâchoire (4, 7; 4', 7'; 4'', 7'') avec les éléments d'actionnement y affectés (3, 6) constituent une pince à saisir le ménisque.
